# EUROPÄISCHE PATENTANMELDUNG

(11) **EP 2 708 203 A1**
(43) Veröffentlichungstag der Anmeldung: **19.03.2014**
(21) Anmeldenummer: 13183130.7
(22) Anmeldetag: 05.09.2013
(51) Int. Cl.: A61B 19/02, A61L 2/26

(54) **Halterung zur Aufnahme von Werkzeugen und Instrumenten aus der Medizintechnik**

(30) Priorität: 12.09.2012 DE 102012108533
(71) Anmelder: HUPFER Metallwerke GmbH & Co. KG, 48653 Coesfeld (DE)
(72) Erfinder: Lanwer, Thorsten, 48653 Coesfeld (DE)
(74) Vertreter: Albrecht, Rainer Harald

(57) **Zusammenfassung**

Die Erfindung betrifft eine Halterung zur Aufnahme von Werkzeugen und Instrumenten aus der Medizintechnik mit einem Träger (1) und ein aus einem elastomeren Material bestehenden biegsamen Leisten (2), die einen Rand mit mindestens einer Vertiefung (3) zur Aufnahme eines Gegenstandes aufweist und an dem Träger (1) lösbar befestigt ist. An den Träger sind seitlich abstehende Zapfen (4) angeordnet. Die biegsame Leiste (2) enthält zugeordnete Löcher (5) und ist auf die Zapfen (4) aufsteckbar. Erfindungsgemäß besteht der Träger (1) aus einem einstückigen Blechprofil, welches einen horizontalen Basisschenkel (6) zur Anlage und zur Befestigung an einem Transportteil, einen rechtwinklig anschließenden Steg (7) und einen abgewinkelten freien Schenkel (8) aufweist. Die Zapfen zur Befestigung der biegsamen Leiste sind an dem vertikalen Steg (7) angeordnet. Der freie Schenkel (8) des Blechprofils ist als Kennzeichnungsfeld mit wasser- und wischfest angeordneten Zeichen oder Symbolen (9) versehen.

## Beschreibung

Die Erfindung betrifft eine Halterung zur Aufnahme von Werkzeugen und Instrumenten aus der Medizintechnik mit einem Träger und einer aus einem elastomeren Material bestehenden biegsamen Leiste. Die Leiste weist einen Rand mit mindestens einer Vertiefung zur Aufnahme eines Gegenstandes auf und ist an dem Träger lösbar befestigt.

Derartige Halterungen werden verwendet, um Werkzeuge und Instrumente aus der Medizintechnik (medizinische Instrumente) an einem Transportteil sicher und gleichzeitig leicht lösbar zu befestigen. Als Transportteile werden beispielsweise Drahtkörbe und Siebschalen verwendet, mit welchen die medizinischen Instrumente Reinigungs-, Desinfektions- oder Sterilisationsein-richtungen zugeführt und für die Benutzung bereitgestellt werden können. Zweckmäßigerweise lassen sich die Halterungen variabel an die Geometrien verschiedener Transportteile anpassen. Gleichzeitig müssen Sie auch so ausgelegt sein, dass sie den teilweise extremen Bedingungen von Reinigung und/oder Sterilisation standhalten können und ihre Formgebung eine vollständige, möglichst einfache und automatische Reinigung ermöglicht. Die Verwendung einer biegsamen Leiste mit Aussparungen zur Lagerung der medizinischen Instrumente erlaubt eine sichere und schonende Fixierung und kann zumindest in gewissen Grenzen auch Vibrations- und schockabsorbierend wirken.

Eine Halterung mit den eingangs beschriebenen Merkmalen zur Aufnahme von medizinischen Instrumenten ist aus der DE 20 2005 016 771 U1 bekannt. Die bekannte Ausführung weist einen als U-förmigen Drahtbügel ausgebildeten Träger auf, an dem Zapfen angeordnet sind. An dem Träger ist eine aus einem Silikonelastomer gebildete biegsame Leiste lösbar befestigbar, welche auf die Zapfen aufsteckbar ist. Die biegsame Leiste weist wenigstens eine Vertiefung zur Aufnahme eines Gegenstandes auf. Ferner ist eine Arretierung der Leiste am Träger beschrieben, bei der die Zapfen zugeordnete Aussparungen am Träger durchgreifen und in einem über die Dicke des Trägers hinausragenden Bereich Verdickungen aufweisen.

Bei der bekannten Halterung ist nachteilig, dass einige Bereiche zwischen Träger und Leiste nur sehr schwer von Reinigungsmittel erreicht werden können und auch nach einer Reinigung oder Sterilisation nur schlecht getrocknet werden können. Eine Ansammlung von Rückständen an schwer zugänglichen Stellen ist die Folge, welche in dem hygienisch hochsensiblen Anwendungsbereich nicht tolerierbar ist. Weiterhin eignet sich die bekannte Kombination aus einem als U-förmiges Drahtteil ausgebildeten Träger und einer aus einem Silikonelastomer gebildeten Leiste nicht, um Beschriftungen anzubringen. Diese sind auf Drahtteilen sehr schwer lesbar und auch in ihrer Größe stark eingeschränkt. Eine Kennzeichnung der elastomeren Leiste kommt aus mehreren Gründen nicht in Betracht. Eine Bedruckung ist aufgrund der schmutzabweisenden Eigenschaften des Werkstoffes, aufgrund der Elastizität und Beweglichkeit des Bauteils und aufgrund der bei der Sterilisation herrschenden Bedingungen nicht praktikabel: Farbstoffe könnten nur sehr schwer auf der Oberfläche zum Haften gebracht werden und würden durch mechanische Beanspruchung im Gebrauch oder durch Wärme und chemische Zusätze bei der Sterilisation wieder gelöst werden. Ein weiterer Nachteil des Standes der Technik besteht darin, dass der Träger für eine bestimmte Geometrie gefertigt werden muss. Insbesondere eine nachträgliche Änderung der Länge ist nicht mehr möglich.

Der Erfindung liegt die Aufgabe zugrunde eine Halterung anzugeben, die effizient gereinigt und sterilisiert werden kann und sich in besonders einfacher und flexibler Weise herstellen, montieren und beschriften lässt.

Gegenstand der Erfindung und Lösung dieser Aufgabe ist eine Halterung gemäß Anspruch 1.

Zum grundsätzlichen Aufbau der Halterung zur Aufnahme von Werkzeugen und Instrumenten aus der Medizintechnik (medizinischen Instrumenten) gehört ein Träger und eine aus einem elastomeren Material bestehende biegsame Leiste, die einen Rand mit mindestens einer Vertiefung zur Aufnahme eines Gegenstandes aufweist. Bei dem Gegenstand kann es sich insbesondere um ein medizinisches Instrument, beispielsweise Operationsbesteck, handeln. Für das elastomere Material kommen vor allem solche Materialien in Frage, welche gegenüber den thermischen und chemischen Einwirkungen eines Sterilisations-prozesses inert sind. Hierfür seien beispielsweise Silikonelastomere oder silikonelastomerhaltige Kunststoffmischungen genannt. Die Form der Aussparung ist zweckmäßig an die Form des aufzunehmenden medizinischen Instruments angepasst. Dabei können an der Berandung der Aussparung Vorsprünge und/oder Zähne vorgesehen sein, welche bei annähernd gleichbleibender Haltewirkung die Auflagefläche minimieren und so das Potential für Schmutz- und/oder Feuchtigkeitsansammlungen reduzieren. Die biegsame Leiste ist an dem Träger lösbar befestig. Sie weist Löcher auf und ist auf Zapfen aufsteckbar, die an dem Träger seitlich abstehend angeordnet sind. Die Zapfen sind metallisch und zylindrisch geformt und können durch Schweißen oder Löten an dem Träger befestigt sein.

Die biegsame Leiste kann zu Reinigungszwecken leicht und ohne Werkzeug von den Zapfen des Trägers abgezogen werden, ohne dass zuvor der Träger an einem Transportteil ausgebaut werden muss. Das Abnehmen der flexiblen Leiste ist insbesondere auch dann problemlos möglich, wenn die Halterung auf dem Boden einer Siebschale oder eines Siebkorbes befestigt ist und sich an beiden Enden bis zu Wandflächen der Siebschale bzw. des Siebkorbes erstreckt.

Erfindungsgemäß besteht der Träger aus einem einstückigen Blechprofil, welches einen horizontalen Basisschenkel zur Anlage und zur Befestigung an einem Transportteil, einen rechtwinklig anschließenden Steg und einen abgewinkelten freien Schenkel aufweist. Dabei sind die Zapfen zur Befestigung der biegsamen Leiste an dem vertikalen Steg angeordnet und ist der freie Schenkel als Kennzeichnungsfeld mit Wasser- und wischfest angeordneten Zeichen oder Symbolen versehen. Vorzugsweise besteht das Blechprofil aus einem rostfreien Stahl oder einem anderen korrosionsbeständigen Metall. Durch die erfindungsgemäße Gestaltung lässt sich der Träger als Endlosprofil herstellen, welches vor dem Gebrauch auf die benötigte Länge zugeschnitten wird. Auch ist ein nachträgliches Einkürzen der gesamten Halterung jederzeit möglich, solange an dem zu verwendenden Stück danach noch ein Zapfen/Loch-Paar verbleibt. Der freie Schenkel stellt ein großes Kennzeichnungsfeld dar, auf dem die Vertiefungen der biegsamen Leiste übersichtlich und dauerhaft bezeichnet werden können. Auch sind durch die Verwendung eines Blechprofiles Formgebungen des Trägers möglich, die eine gründliche und einfachere Reinigung ermöglichen. Einige mögliche Varianten sind als bevorzugte Ausführungsformen Gegenstand der Unteransprüche.

In einer dieser bevorzugten Ausgestaltungen ist das Blechprofil im Querschnitt U-förmig ausgebildet, wobei der Basisschenkel eine größere Breite aufweist als das Kennzeichnungsfeld und die flexible Leiste auf dem Basisschenkel abgestützt ist. Die Zapfen erstrecken sich durch den U-förmigen Raum des Blechprofils und weisen eine Länge auf, die mindestens der Breite des Basisschenkels entspricht. In diesem Zusammenhang bedeutet "U-förmig", dass der freie Schenkel vom vertikalen Steg aus in Richtung des Basisschenkels weggebogen ist, wodurch ein von Basisschenkel, vertikalem Steg und freiem Schenkel an drei Seiten umschlossener Raum entsteht. Der Winkel zwischen dem vertikalen Steg und dem freien Schenkel ist nicht auf 90° beschränkt. Durch die hier beschriebene Anordnung wird verhindert, dass sich eine Seitenfläche der biegsamen Leiste an eine Innenfläche des vertikalen Steges anlegt und einen schwer zugänglichen und schwer zu trocknenden Bereich der Halterung bildet.

Vorzugsweise enthält der vertikale Steg des Blechprofils großflächige Öffnungen, die sich vom Basisschenkel bis zum Kennzeichnungsfeld erstrecken. Durch diese Öffnungen kann Reinigungsflüssigkeit zuverlässig in den Raum zwischen Blechprofil und biegsamer Leiste ein- und auch wieder austreten, wobei sich die Reinigungsflüssigkeit auch gleichmäßig über die gesamte Länge des Blechprofils verteilen kann.

Vorzugsweise sind die Öffnungen dreieckförmig ausgebildet, wobei die Spitzen der dreieckförmigen Öffnungen dem Basisschenkel des Blechprofils zugewandt sind. Die dreieckförmige Form weist vielerlei Vorzüge auf: Dreiecke sind eine sehr einfache geometrische Figur, welche sich mit Standardwerkzeugen und ohne großen Aufwand herstellen lassen. Die Anordnung einer Spitze am unteren Rand des vertikalen Steges lässt im Übergangsbereich zum Basisschenkel einen Großteil des Materials stehen, wodurch eine besonders steife und stabile Verbindung der beiden Flächen und somit eine zuverlässige Lagerung der Zapfen besteht. Die Kante zum freien Schenkel hingegen wird durch großflächigen Materialaustrag geschwächt, so dass zum Abkanten ein flexibles Freiformbiegeverfahren verwendet werden kann. Dies erlaubt eine verhältnismäßig problemlose Variation des Biegewinkels bei der Produktion des Profils. Bei gering gewähltem Abstand der dreieckförmigen Öffnung lässt sich eine Durchsetzung der Aussparungen von annähernd 50 %, mindestens jedoch 40 % erreichen. Auch unter reinigungstechnischen Gesichtspunkten ist diese Form vorteilhaft. Die Öffnungen sind im oberen Bereich größer, so dass dort besonders viel Reinigungsflüssigkeit eintreten kann. Von dort kann sie selbstständig nach unten fließen und Bereiche erreichen, welche durch die unteren Schenkel der Dreiecke verdeckt sind.

In einer besonders vorteilhaften Ausgestaltung ist die biegsame Leiste an ihrer Unterkante an dem Basisschenkel des Trägers formschlüssig fixiert. Hierdurch kann ein Herunterrutschen zuverlässig ausgeschlossen werden. Weiterhin lässt sich der Abstand der biegsamen Leiste zum vertikalen Steg und zum freien Schenkel des Blechprofils durch die Ausgestaltung der formschlüssigen Fixierung einstellen, wodurch die Reinigungseigenschaften weiter beeinflusst und verbessert werden können.

Vorzugsweise weist die biegsame Leiste an ihrer Unterkante eine Nut auf. An dem Basisschenkel des Blechprofils sind dann Erhebungen angeformt, die in die Nut der biegsamen Leiste eingreifen. Derartige Erhebungen können beispielsweise durch Körnung des Basisschenkels oder durch Auftrag von Löt- oder Schweißpunkten gebildet sein.

Zweckmäßigerweise durchfassen die Zapfen des Trägers die korrespondierenden Löcher der biegsamen Leiste und stehen an der Außenfläche der biegsamen Leiste vor. Hierdurch ist gewährleistet, dass die Löcher in der biegsamen Leiste vollständig durch die Zapfen ausgefüllt sind und sich darin keine Rückstände ansammeln können. Die Zapfen sind vorzugsweise als zylindrische Stifte ausgebildet, die über ihre gesamte Länge einen konstanten Durchmesser aufweisen. Die zugeordneten Löcher der biegsamen Leiste weisen ein Untermaß auf, wodurch die Leiste mit einem Klemmsitz auf den Zapfen fixiert ist.

Vorzugsweise ist zwischen dem freien Schenkel und der auf die Zapfen des Trägers aufgesteckten biegsamen Leiste ein Spalt vorhanden. Der Spalt unterstützt den Durchfluss von Reinigungsflüssigkeit durch den zwischen biegsamer Leiste und vertikalem Steg gelegenen Raum und wirkt sich positiv auf die Reinigungseigenschaften aus. Um dies weiter zu fördern, kann der freie Rand des Kennzeichnungsfeldes zusätzlich ein Profil aus Vorsprüngen und Rücksprüngen aufweisen, wobei zwischen den Rücksprüngen und der auf die Zapfen des Trägers aufgesteckten biegsamen Leiste spaltförmige Öffnungen für den Durchfluss von Spülflüssigkeit resultieren. Hierdurch ist auch eine großflächige Zuführung der Spülflüssigkeit von oben möglich.

Bei einer U-förmigen Ausgestaltung schließt das Kennzeichnungsfeld bevorzugt unter einem Winkel von 90° bis 120° an den vertikalen Steg des Blechprofils an. Diese leichte Schrägstellung bewirkt ein einfaches Ablaufen der Spülflüssigkeit, ohne darunter einen aufgrund seiner Spitzwinkligkeit schwierig zu reinigenden Raum zu schaffen. Außerdem begünstigt die leichte Neigung die Lesbarkeit der Beschriftung.

In einer alternativen Ausgestaltung ist das Blechprofil im Querschnitt Z-förmig ausgebildet, wobei das Kennzeichnungsfeld unter einem Winkel von 60° bis 90° an den vertikalen Steg des Blechprofils anschließt. Diese Ausgestaltung kann je nach Einbausituation vorteilhaft sein. Sie resultiert in einem besonders geringen Abstand zwischen vertikalem Steg und der biegsamen Leiste. Um dennoch eine gute Spülfähigkeit zu erhalten, können an den Zapfen Abstandhalter vorgesehen sein oder die zuvor beschriebenen Maßnahmen zur unterseitigen Fixierung, der Leiste so ausgebildet werden, dass der Steg des Trägers und die Leiste nicht flächig aneinander anliegen.

Besonders bevorzugt sind den Zeichen und/oder Symbolen des Kennzeichnungsfeldes Vertiefungen der biegsamen Leiste räumlich zugeordnet. Die Vorteile einer solchen Ausgestaltung sind vielfältig. Durch feste Zuordnungen von medizinischem Instrument und Vertiefung können Betriebsabläufe verbessert und Gefahrenpotentiale aufgrund falscher Zuordnung von Instrumenten verringert werden.

Bevorzugt werden die Zeichen und/oder Symbole durch Lasergravur erzeugt. Diese Methode stellt eine sehr flexible und schnelle Möglichkeit dar, beliebige Formen auf metallische Oberflächen aufzubringen. Insbesondere können auch Instrumente, für die die Vertiefungen der biegsamen Leiste bestimmt sind, auf der Fläche des Kennzeichnungsfeldes graphisch dargestellt werden. Die durch die Laserbestrahlung veränderten Bereiche weisen eine glatte Oberfläche auf und sind nicht übermäßig für Schmutzbesatz anfällig.

Zweckmäßigerweise ist der Basisschenkel des Trägers zur Befestigung auf einer Siebschale mit Montageöffnungen für Schrauben oder Nieten versehen. Um eine größtmögliche Flexibilität zu erreichen kann der Basisschenkel nahezu flächendeckend mit einer großen Anzahl von Öffnung durchsetzt sein. Hierbei können nicht für die Befestigung genutzte Öffnungen gleichzeitig dem Abfluss und/oder der Zuführung von Spülflüssigkeit dienen.

Die Erfindung soll anhand der folgenden Zeichnungen erläutert werden. Es zeigen schematisch
- **Fig. 1**: die perspektivische Darstellung einer erfindungsgemäßen Halterung im montierten Zustand,
- **Fig. 2**: die Stirnansicht einer erfindungsgemäßen Halterung mit U-förmigem Blechprofil,
- **Fig. 3**: die Halterung aus Fig. 1 im demontierten Zustand,
- **Fig. 4**: den Gegenstand der Fig. 3 aus einem anderem Blickwinkel,
- **Fig. 5a, 5b**: eine Ausführungsvariante der erfindungsgemäßen Halterung im montierten und im getrennten Zustand,
- **Fig. 6a, 6b**: eine andere Ausführungsform einer erfindungsgemäßen Halterung in perspektivischer Darstellung und als Stirnansicht und
- **Fig. 7a, 7b**: ein weiteres Ausführungsbeispiel einer erfindungsgemäßen Halterung in perspektivischer Darstellung und als Stirnansicht.

Zum grundsätzlichen Aufbau der in den Figuren dargestellten Halterungen zur Aufnahme von Werkzeugen und Instrumenten aus der Medizintechnik gehören ein Träger 1 und eine aus einem elastomeren Material bestehende biegsame Leiste 2. Die Leiste 2 weist einen Rand mit mindestens einer Vertiefung 3 zur Aufnahme eines Gegenstandes auf und ist am Träger 1 lösbar befestigt. An dem Träger 1 sind seitlich abstehende Zapfen 4 angeordnet, wobei die biegsame Leiste 2 zugeordnete Löcher 5 enthält und auf die Zapfen 4 aufsteckbar ist. Erfindungsgemäß besteht der Träger 1 aus einem einstückigen Blechprofil, welches einen horizontalen Basisschenkel 6 zur Anlage und zur Befestigung an einem Transportteil, einen rechtwinklig anschließenden Steg 7 und einen abgewinkelten freien Schenkel 8 aufweist. Die Zapfen 4 zur Befestigung der biegsamen Leiste 2 sind am vertikalen Steg 7 angeordnet. Der freie Schenkel 8 ist als Kennzeichnungsfeld mit Wasser- und wischfest angeordneten Zeichen oder Symbolen 9 versehen.

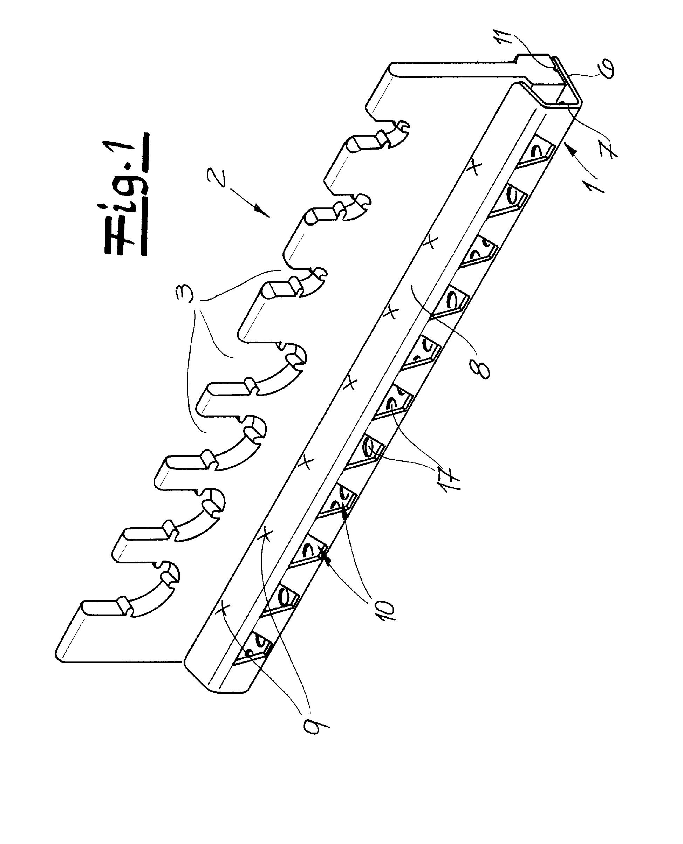

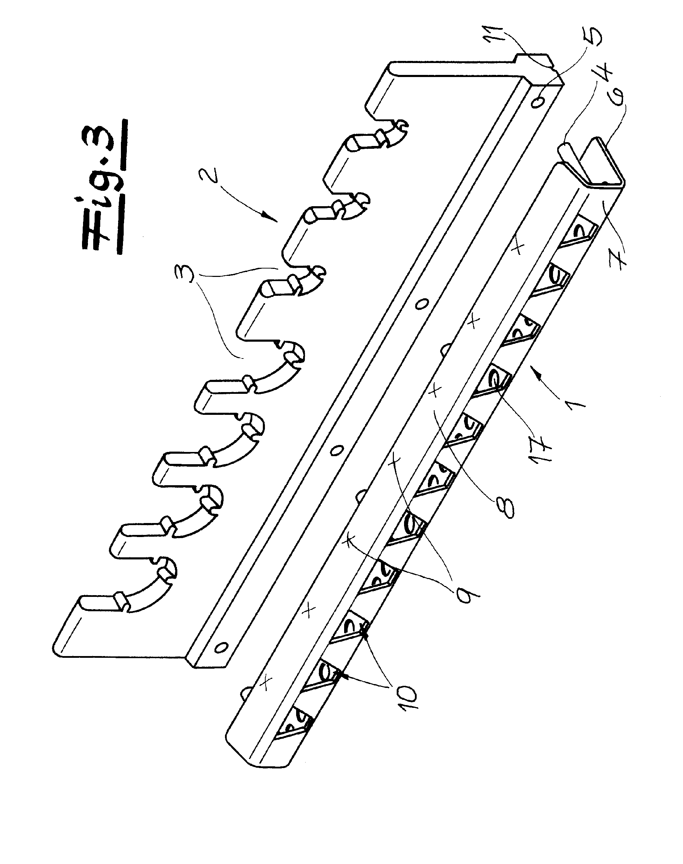

Bei dem in den Fig. 1 bis 5b dargestellten Ausführungsformen ist das Blechprofil im Querschnitt U-förmig ausgebildet. Dabei weist der Basisschenkel 6 eine größere Breite auf als das Kennzeichnungsfeld 8. Die flexible Leiste 2 ist auf dem Basisschenkel abgestützt, und die Zapfen 4 erstrecken sich durch den U-förmigen Raum des Blechprofils. Sie weisen eine Länge auf, die mindestens der Breite des Basisschenkels 6 entspricht.

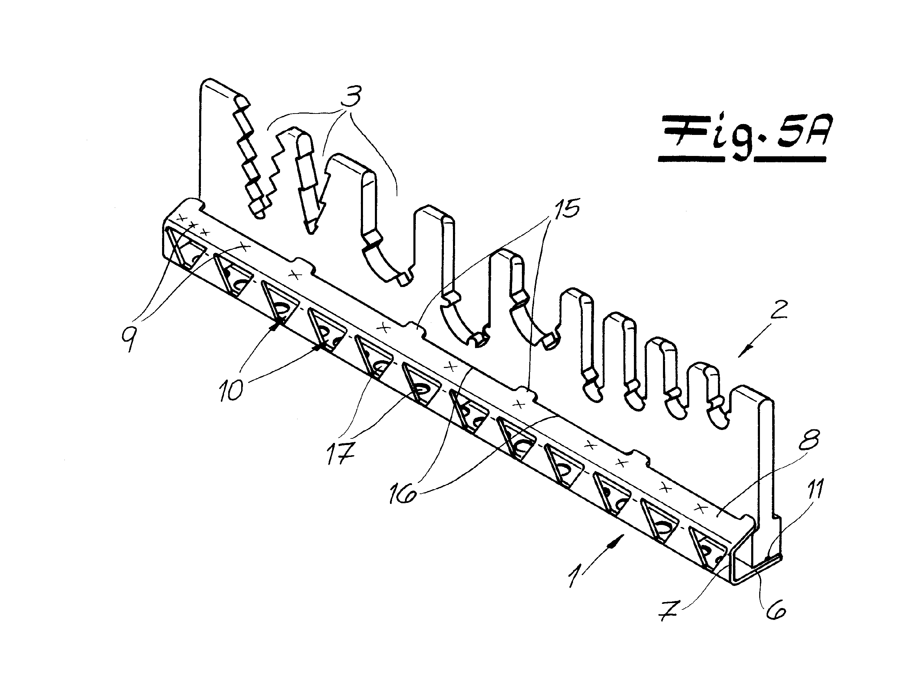

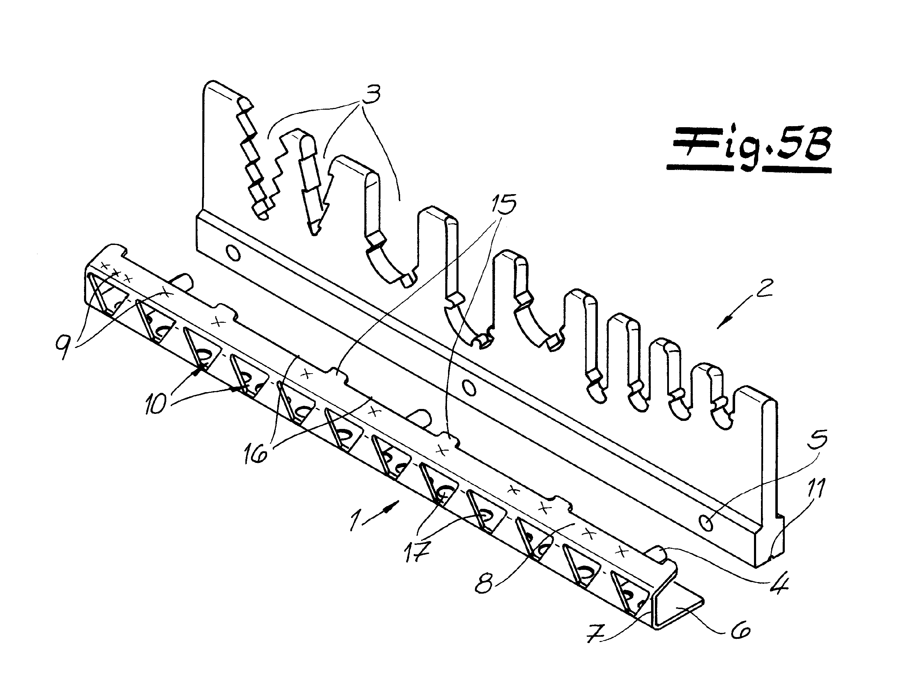

Der Steg 7 enthält gemäß den Darstellungen in den Fig. 1, 5a und 5b großflächige Öffnungen 10, die sich vom Basisschenkel 6 bis zum Kennzeichnungsfeld 8 erstrecken. Diese sind dreieckförmig ausgebildet, wobei die Spitzen der dreieckförmigen Öffnungen 10 dem Basisschenkel 6 des Blechprofils zugewandt sind. Die beiden anderen Ecken der Dreiecke sind am Übergang zum freien Schenkel 8 angeordnet.

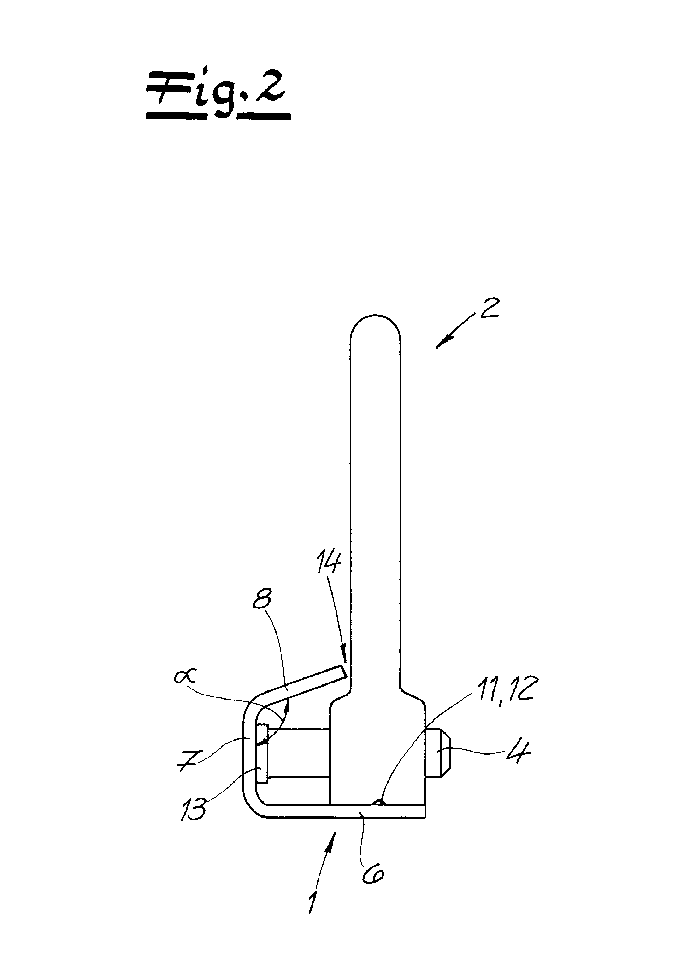

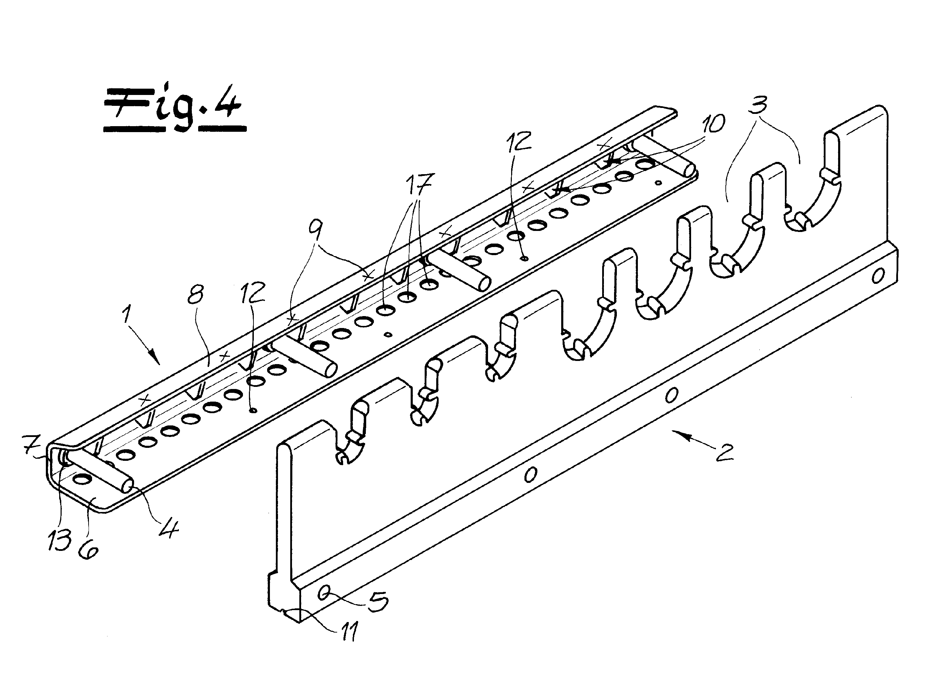

In den Querschnittzeichungen der Fig. 2, 6b und 7b ist dargestellt, dass die biegsame Leiste 2 an Ihrer Unterkante an dem Basisschenkel 6 des Trägers 1 formschlüssig fixiert ist. Dazu weist die biegsame Leiste 2 an ihrer Unterkante eine Nut 11 auf und sind an dem Basisschenkel des Blechprofils Erhebungen 12 angeformt, die in die Nut 11 der biegsamen Leiste 2 eingreifen. Die Erhebungen 12 sind in der Fig. 4 als Körnungspunkte dargestellt.

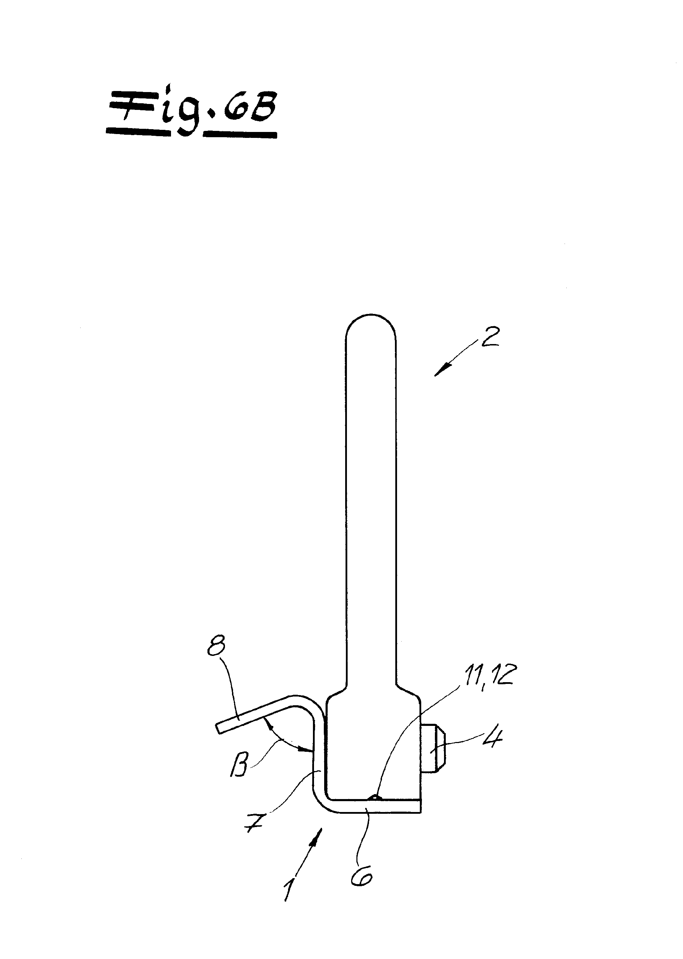

Ferner durchfassen die Zapfen 4 des Trägers 1 die korrespondierenden Löcher 5 der biegsamen Leiste 2 und stehen an der Außenfläche der biegsamen Leiste 2 vor. In Fig. 6b sind die Zapfen 4 als zylindrische Stifte ausgebildet, die über ihre gesamte Länge einen konstanten Durchmesser aufweisen. Die zugeordneten Löcher 5 der biegsamen Leiste 2 weisen ein Untermaß auf, wodurch die Leiste 2 mit einem Klemmsitz auf den Zapfen 4 fixiert ist.

Die Zapfen 4 sind als zylindrische Stifte ausgebildet, die über ihre gesamte Länge einen konstanten Durchmesser aufweisen. Sie sind mit einer Kontaktstelle 13 mit dem vertikalen Steg 7 verbunden. Die zugeordneten Löcher 5 der biegsamen Leiste 2 weisen ein Untermaß auf. Dadurch ist die Leiste 2 mit einem Klemmsitz auf dem Zapfen 4 fixiert.

In Fig. 2 ist zwischen dem freien Schenkel 8 und der auf dem Zapfen 4 des Trägers 1 aufgesteckten biegsamen Leiste 2 ein Spalt 14 vorhanden.

Die Fig. 5a und 5b betreffen eine Ausführungsform, bei dem der freie Rand des Kennzeichnungsfeldes 8 ein Profil aus Vorsprüngen 15 und Rücksprüngen 16 aufweist. Dabei resultieren zwischen den Rücksprüngen 16 und der auf die Zapfen 4 des Trägers 1 aufgesteckten Leiste 2 spaltförmige Öffnungen für den Durchfluss von Spülflüssigkeit.

Bei den Ausführungen mit einem U-förmigen Blechprofil schließt das Kennzeichnungsfeld 8 unter einem Winkel α (s. Fig. 2) von 90° bis 120° an den vertikalen Steg 7 des Blechprofils an.

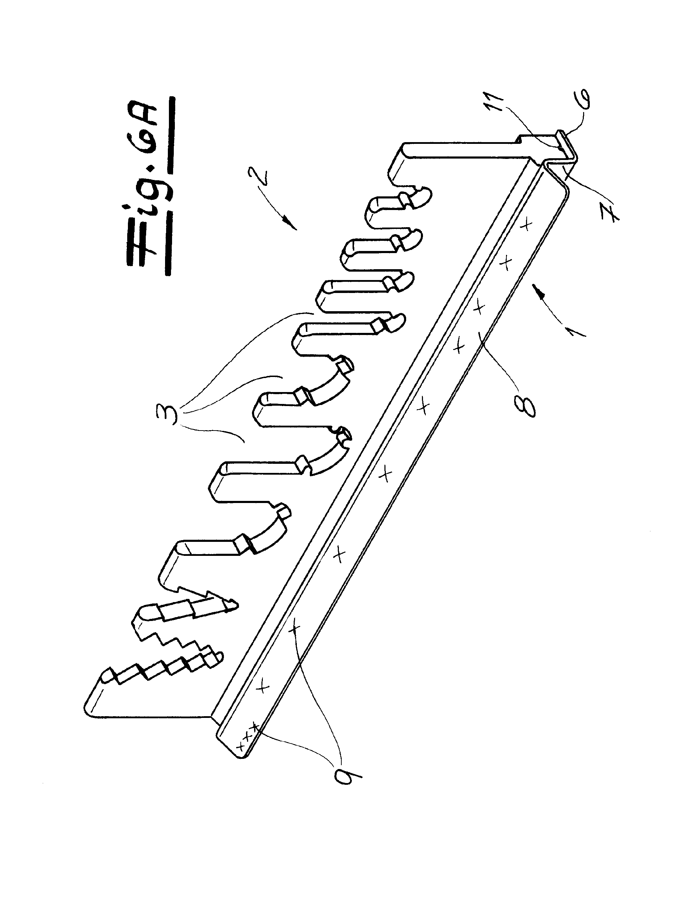

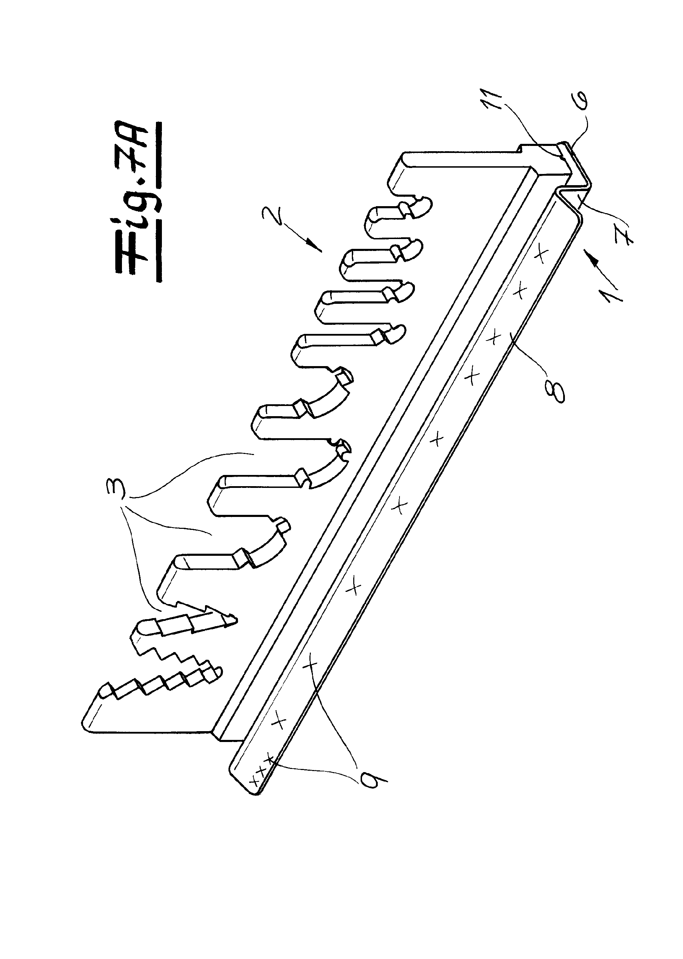

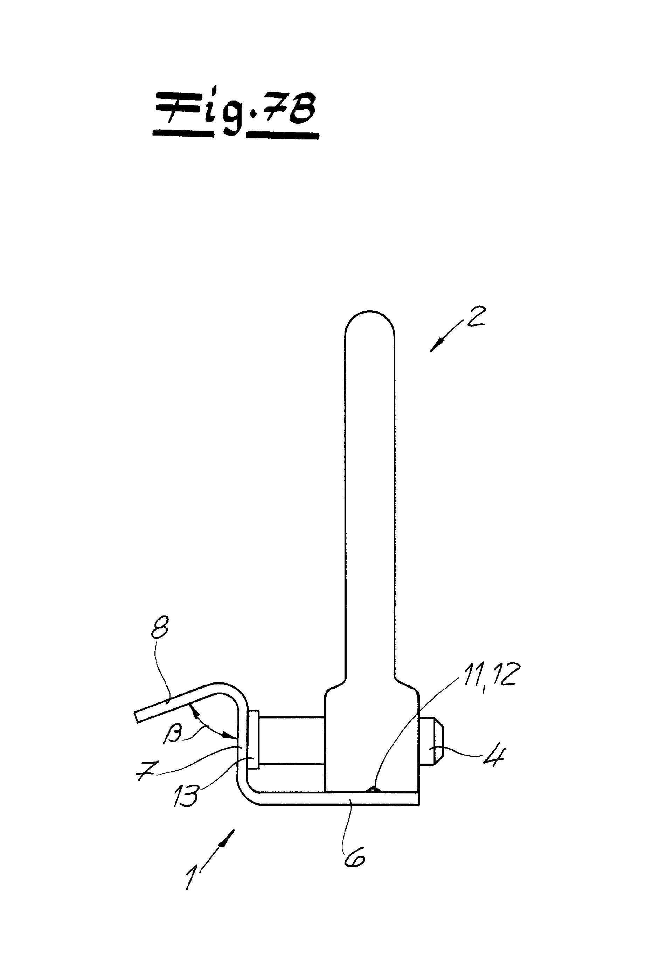

In den in den Fig. 6a bis 7b dargestellten Ausführungsformen ist das Blechprofil im Querschnitt Z-förmig ausgebildet, wobei das Kennzeichnungsfeld 8 unter einem Winkel β (s. Fig. 6b, 7b) von 60° bis 90° an den vertikalen Steg 7 des Blechprofils anschließt. Die Fig. 6a und 7a zeigen überdies alternative Ausgestaltungen der Vertiefungen 3 der biegsamen Leiste 2. Diese variieren bezüglich ihrer Breite, Einschnitttiefe und Ausgestaltung der Berandung. Dazu zählen gesägte Ränder und Ränder mit Auflagepunkten, welche sowohl abgerundet als auch spitz ausgeführt sein können.

Die Ausführung in den Fig. 6a und 6b ist dadurch gekennzeichnet, dass die biegsame Leiste 2 unmittelbar an dem vertikalen Steg 7 des Blechprofils anliegt. In der alternativen Ausführung (Fig. 7a und 7b) weisen die Zapfen 4 an ihrer dem Steg 7 zugewandten Seite eine Verdickung 13 auf, welche einen minimalen Abstand zwischen Steg 7 und biegsamer Leiste 2 festlegt.

Bei allen Beispielen sind Zeichen und/oder Symbole 9 des Kennzeichnungsfeldes 8 den Vertiefungen 3 der biegsamen Leiste 2 räumlich zugeordnet. Im Beispiel sind die Zeichen und/oder Symbole 9 durch Lasergravur erzeugt worden.

In Fig. 4 ist überdies sichtbar, dass der Basisschenkel 6 des Trägers 1 zur Befestigung auf einer Siebschale mit Montageöffnungen 17 für Schrauben oder Nieten versehen ist. Nicht genutzte Montageöffnungen 17 können als zusätzliche Zu- oder Abflussöffnungen für Spülflüssigkeit dienen.

## Patentansprüche

1. Halterung zur Aufnahme von Werkzeugen und Instrumenten aus der Medizintechnik mit einem Träger (1) und einer aus einem elastomeren Material bestehenden biegsamen Leiste (2), die einen Rand mit mindestens einer Vertiefung (3) zur Aufnahme eines Gegenstandes aufweist und an dem Träger (1) lösbar befestigt ist, wobei an dem Träger seitlich abstehende Zapfen (4) angeordnet sind und wobei die biegsame Leiste (2) zugeordnete Löcher (5) enthält und auf die Zapfen (4) aufsteckbar ist, **dadurch gekennzeichnet, dass** der Träger (1) aus einem einstückigen Blechprofil besteht, welches einen horizontalen Basisschenkel (6) zur Anlage und zur Befestigung an einem Transportteil, einen rechtwinklig anschließenden Steg (7) und einen abgewinkelten freien Schenkel (8) aufweist, wobei an dem vertikalen Steg (7) die Zapfen zur Befestigung der biegsamen Leiste angeordnet sind und wobei der freie Schenkel (8) als Kennzeichnungsfeld mit wasser- und wischfest angeordneten Zeichen oder Symbolen (9) versehen ist.

2. Halterung nach Anspruch 1, **dadurch gekennzeichnet, dass** das Blechprofil im Querschnitt U-förmig ausgebildet ist, wobei der Basisschenkel (6) eine größere Breite aufweist als das Kennzeichnungsfeld (8), dass die flexible Leiste (2) auf dem Basisschenkel (6) abgestützt ist und dass die Zapfen (4) sich durch den U-förmigen Raum des Blechprofils erstrecken und eine Länge aufweisen, die mindestens der Breite des Basisschenkels (6) entspricht.

3. Halterung nach Anspruch 2, **dadurch gekennzeichnet, dass** der Steg (7) des Blechprofils großflächige Öffnungen (10) enthält, die sich vom Basisschenkel (6) bis zum Kennzeichnungsfeld (8) erstrecken.

4. Halterung nach Anspruch 3, **dadurch gekennzeichnet, dass** die Öffnungen (10) dreieckförmig ausgebildet sind, wobei die Spitzen der dreieckförmigen Öffnungen (10) dem Basisschenkel (6) des Blechprofils zugewandt sind.

5. Halterung nach einem der Ansprüche 2 bis 4, **dadurch gekennzeichnet, dass** die biegsame Leiste (2) an ihrer Unterkante an dem Basisschenkel (6) des Trägers (1) formschlüssig fixiert ist.

6. Halterung nach Anspruch 5, **dadurch gekennzeichnet, dass** die biegsame Leiste (2) an ihrer Unterkante eine Nut (11) aufweist und dass an dem Basisschenkel (6) des Blechprofils Erhebungen (12) angeformt sind, die in die Nut (11) der biegsamen Leiste (2) eingreifen.

7. Halterung nach einem der Ansprüche 1 bis 6, **dadurch gekennzeichnet, dass** die Zapfen (4) des Trägers (1) die korrespondierenden Löcher (5) der biegsamen Leiste (2) durchfassen und an der Außenfläche der biegsamen Leiste (2) vorstehen.

8. Halterung nach einem der Ansprüche 1 bis 7, **dadurch gekennzeichnet, dass** die Zapfen (4) als zylindrische Stifte ausgebildet sind, die über ihre gesamte Länge einen konstanten Durchmesser aufweisen, und dass die zugeordneten Löcher (5) der biegsamen Leiste (2) ein Untermaß aufweisen und dadurch die Leiste (2) mit einem Klemmsitz auf den Zapfen (4) fixiert ist.

9. Halterung nach einem der Ansprüche 1 bis 7, **dadurch gekennzeichnet, dass** die Zapfen (4) in einem dem vertikalen Steg (7) abgewandten Bereich als zylindrische Stifte ausgebildet sind, die in diesem Bereich einen konstanten Durchmesser aufweisen, und dass die Zapfen (4) in einem zweiten dem vertikalen Steg (7) zugewandten Bereich eine Verdickung aufweisen, die einen Anschlag für die biegsame Leiste (2) bildet und einen minimalen Abstand zwischen Steg (7) und Leiste (2) festgelegt.

10. Halterung nach einem der Ansprüche 2 bis 9, **dadurch gekennzeichnet, dass** zwischen dem freien Schenkel (8) und der auf die Zapfen (4) des Trägers (1) aufgesteckten biegsamen Leiste (2) ein Spalt (14) vorhanden ist.

11. Halterung nach einem der Ansprüche 2 bis 9, **dadurch gekennzeichnet, dass** der freie Rand des Kennzeichnungsfeldes (8) ein Profil aus Vorsprüngen (15) und Rücksprüngen (16) aufweist, wobei zwischen den Rücksprüngen (16) und der auf die Zapfen (4) des Trägers (1) aufgesteckten Leiste (2) spaltförmige Öffnungen für den Durchdruck von Spülflüssigkeit resultieren.

12. Halterung nach einem der Ansprüche 2 bis 11, **dadurch gekennzeichnet, dass** das Kennzeichnungsfeld (8) unter einem Winkel (α) von 90° bis 120° an dem vertikalen Steg (7) des Blechprofils anschließt.

13. Halterung nach Anspruch 1, **dadurch gekennzeichnet, dass** das Blechprofil im Querschnitt Z-förmig ausgebildet ist, wobei das Kennzeichnungsfeld (8) unter einem Winkel (β) von 60° bis 90° an den vertikalen Steg (7) des Blechprofils anschließt.

14. Halterung nach Anspruch 13, **dadurch gekennzeichnet, dass** die Zapfen (4) an ihrer dem Steg (7) zugewandten Seite eine Verdickung aufweisen, welche einen minimalen Abstand zwischen Steg (7) und biegsamer Leiste (2) festlegt.

15. Halterung nach einem der Ansprüche 1 bis 14, **dadurch gekennzeichnet, dass** die Zeichen und/oder Symbole (9) des Kennzeichnungsfeldes (8) Vertiefungen (3) der biegsamen Leiste (2) räumlich zugeordnet sind.

16. Halterung nach einem der Ansprüche 1 bis 15, **dadurch gekennzeichnet, dass** die Zeichen und/oder Symbole (9) durch Lasergravur erzeugt worden sind.

17. Halterung nach einem der Ansprüche 1 bis 16, **dadurch gekennzeichnet, dass** der Basisschenkel (6) des Trägers (1) zur Befestigung auf einer Siebschale mit Montageöffnungen (17) für Schrauben oder Nieten versehen ist.
